# EUROPÄISCHE PATENTSCHRIFT

(11) **EP 0 339 450 B1**
(45) Veröffentlichungstag und Bekanntmachung des Hinweises auf die Patenterteilung: **22.02.1995**
(21) Anmeldenummer: 89106993.2
(22) Anmeldetag: 19.04.1989
(51) Int. Cl.: G01N 33/543, G01N 33/52

(54) **Testträger für die Analyse einer Probenflüssigkeit, Verfahren zur Durchführung einer solchen Analyse und Herstellungsverfahren**
Test strip for the analysis of a sample liquid, process for conducting such an analysis and process for its preparation
Support d'essai pour l'analyse d'un liquide d'échantillons, procédé de réalisation d'une telle analyse et procédé de préparation

(30) Priorität: 28.04.1988 DE 3814370
(43) Veröffentlichungstag der Anmeldung: 02.11.1989
(73) Patentinhaber: BOEHRINGER MANNHEIM GMBH, 68298 Mannheim (DE)
(72) Erfinder: Wilk, Hans-Erich, Dr., D-6143 Lorsch (DE); Münter, Karin, Dr., D-6800 Mannheim 1 (DE); Lerch, Rolf, Dipl.-Ing., D-6804 Ilvesheim (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 013 156
- EP-A- 0 262 445
- DE-A- 3 624 464

## Beschreibung

Die Erfindung richtet sich auf einen Testträger für die analytische Bestimmung eines Bestandteils einer Probenflüssigkeit mit Hilfe von immunologischen Reagenzien sowie ein dies bezügliches Analyse- und Herstellungsverfahren
Immunologische Verfahren spielen in der Analytik, insbesondere bei der Analyse von Körperflüssigkeiten wie Blut oder Urin eine zunehmende Rolle. Sie zeichnen sich dadurch aus, daß sie hochspezifisch und äusserst empfindlich sind. Die Nachweisverfahren basieren auf der immunologischen Wechselwirkung zwischen spezifisch miteinander bindungsfähigen Bindungspartnern, insbesondere Antigenen (oder Haptenen) und den entsprechenden Antikörpern. Häufig ist der zu bestimmende Bestandteil selbst einer der Bindungspartner. Es gibt jedoch eine Vielzahl von verschiedenen Verfahrensführungen, bei denen teilweise auch mehrere immunologische Bindungsreaktionen zwischen verschiedenen Bindungspartner-Paaren ablaufen.

Durch Markieren eines der Bindungspartner kann ein meßbares Signal erzeugt werden, das dann zur Auswertung herangezogen wird. Man unterscheidet verschiedene immunologische Bestimmungsverfahren nach der gewählten Markierung. Die vorliegende Erfindung richtet sich vor allem auf sogenannte Enzymimmunoassays, bei denen ein Enzym als Markierung verwendet wird. Der Nachweis des Enzyms erfolgt dabei, wie dies auch bei der Enzymdiagnostik üblich ist, indem man das Enzym auf ein Substrat einwirken läßt, welches eine optisch nachweisbare Veränderung erfährt. Meist besteht die optisch nachweisbare Änderung in einem Farbumschlag. Alternativ kann beispielsweise eine Markierung mit einem Fluoreszenzfarbstoff verwendet werden.

Immunologische Nachweisverfahren erfordern meist eine Vielzahl von komplizierten, nacheinander ablaufenden Verfahrensschritten und lange Inkubationszeiten. Sie wurden deswegen lange Zeit ausschließlich naßchemisch manuell oder mit Hilfe aufwendiger Analysegeräte durchgeführt.

In jüngerer Zeit haben für die Analyse von Körperflüssigkeiten sogenannte "trägergebundene Tests" zunehmend an Bedeutung gewonnen. Bei diesen sind Reagenzien in einer oder mehrere Testschichten eines festen Testträgers eingebettet, der mit der Probe in Kontakt gebracht wird. Die Reaktion von Probe und Reagenzien führt üblicherweise zu einem Farbumschlag, der reflexionsphotometrisch ausgewertet wird. Die Testträger und die entsprechenden Auswertegeräte werden insgesamt als Testträger-Analysesysteme bezeichnet.

Testträger-Analysesysteme zeichnen sich vor allem durch eine einfache Handhabung aus. Außerdem sind die Geräte verhältnismäßig kostengünstig, so daß sie auch im Labor des praktizierenden Arztes oder in der Krankenstation aufgestellt werden können.

In Anbetracht dieser Vorteile wurden auch bereits Testträger für immunologische Bestimmungen vorgeschlagen. Damit sind jedoch erhebliche Schwierigkeiten verbunden, weil die auf Testträgern ablaufenden Reaktionen nicht die zahlreichen Manipulationsvorgänge erlauben, die üblicherweise für immunologische Bestimmungen notwendig sind.

In der US-A-4 446 322 ist ein einfacher Aufbau eines solchen Testträgers beschrieben, bei dem zwei oder drei Testschichten derartig übereinander angeordnet sind, daß sie ständig in einem Flüssigkeitsaustausch ermöglichenden Kontakt zueinander stehen. Bei der dreischichtigen Ausführungsform befindet sich in der ersten Schicht ein Enzymkonjugat eines ersten immunologischen Bindungspartners, beispielsweise eines Antikörpers, in der mittleren Schicht ein mit dem ersten Bindungspartner spezifisch bindungsfähiger zweiter Bindungspartner, beispielsweise ein Antigen zu dem Antikörper, in trägerfixierter Form, und in der untersten Schicht ein farbbildendes Substrat für das Enzym.

Der in dieser Druckschrift beschriebene Testablauf ist dem IEMA-Test, der von naßchemischen immunologischen Bestimmungen bekannt ist, nachgebildet: ein in der Probe enthaltenes Antigen soll in der obersten Schicht mit dem Antikörper-Enzym-Konjugat komplexieren. Die Komplexe sowie nichtkomplexiertes überschüssiges Enzymkonjugat gelangt in die mittlere Schicht, wo eine Bindungsreaktion zwischem dem überschüssigen Enzymkonjugat und dem trägerfixierten Antigen stattfindet, so daß das überschüssige Enzymkonjugat fixiert wird (eine solche Schicht wird hier als "Fixierungsschicht" bezeichnet). Die Komplexe können die mittlere Schicht durchwandern und gelangen in die Substratschicht, wo eine entsprechende Farbreaktion stattfinden soll.

Diese einfache Übertragung des naßchemischen Tests auf den Testträger hat sich in der Praxis nicht bewährt. Die Flussigkeit durchströmt den Testträger unkontrolliert, während bei dem naßchemischen Test ein exakter Reaktionsablauf mit sauber getrennten Reaktionsschritten notwendig ist.

Ein anderer mehrschichtiger Aufbau mit festverbundenen Schichten ist in der DE-A-33 29 728 beschrieben. Diese Druckschrift geht auch ausführlich auf mögliche Testschichtstrukturen ein.

Aus der DE-B-34 45 816 ist ein immunologischer Testträger bekannt, bei dem mehrere Testschichten nebeneinander auf einer Basisschicht angeordnet sind. Die Testschichten stehen über ihre Kanten ständig in saugfähigem Kontakt zueinander.

Der Erfindung liegt die Aufgabe zugrunde, Immuntests zur Verfügung zu stellen, die einfach zu handhaben sind und eine hohe Testgenauigkeit erlauben. Der Testträger soll dennoch möglichst einfach aufgebaut und kostengünstig herzustellen sein.

Die Aufgabe wird durch den Testträger nach Anspruch 1 und die Verfahren nach Anspruch 15 und 17 gelöst.

Der bevorzugte Testablauf in dem erfindungsgemäßen Testträger hat eine gewisse Ähnlichkeit zu dem in der US-A-4 446 322 beschriebenen. Soll beispielsweise ein in der Probenflüssigkeit enthaltenes Antigen bestimmt werden, so befindet sich in der Konjugatschicht ein Enzymkonjugat eines entsprechenden Antikörpers. In der Fixierungsschicht befindet sich das Probenantigen oder ein Analogon hierzu (welches mit dem gleichen Antikörper bindet) in trägerfixierter Form.Durch die inder Fixierungsschicht ablaufende Bindungsreaktion wird Konjugat, welches nicht eine Bindung mit dem freien Antigen aus der Probe eingeht, an das trägerfixierte Antigen der Fixierungsschicht gebunden.

Im Unterschied zu dem vorbekannten Testträger wird bei der Erfindung nicht angestrebt, daß in der Konjugatschicht die Bindungsreaktion zwischen dem Konjugat und dem Analyt möglichst vollständig abläuft und die Fixierung des überschüssigen Konjugats beim Eintreten der Probenflüssigkeit in die Fixierungsschicht möglichst schnell und vollständig geschieht.

Dies würde durch einen langsamen Saugvorgang in der Fixierungsschicht gefördert.

Im Rahmen der vorliegenden Erfindung wurde vielmehr festgestellt, daß eine hohe Sauggeschwindigkeit in der Fixierungsschicht anzustreben ist, so daß sich die Flüssigkeit verhältnismäßig schnell in dieser Schicht ausbreitet. Diese für die Ausbreitung der Flüssigkeit benötigte Zeit soll kurz sein im Verhältnis zu der Inkubationszeit der in der Fixierungsschicht ablaufenden immunologischen Bindungsreaktion. Der Begriff "Inkubationszeit" bezeichnet hier die Zeit, in der die Bindungsreaktion praktisch vollständig (zumindest 95%) abläuft. Diese Inkubationszeit ist abhängig vom konkreten Test, d.h. von den Eigenschaften des in dem Testträger fixierten ersten und zweiten Bindungspartners. Für einen bestimmten Testträger ist die notwendige Inkubationszeit für den Ablauf der Bindungsreaktion deswegen für den Fachmann eine bestimmte Größe.

Typischerweise sind mit den heute verfügbaren Antigen-Antikörperpaaren Inkubationszeiten von ein bis drei Minuten ausreichend. In diesem Fall sollte die Ausbreitungszeit der Flüssigkeit in der Fixierungsschicht kleiner als 30 sec, bevorzugt weniger als 20 sec betragen, wobei typische Werte um 10 sec liegen. Die Ausbreitungszeit sollte höchstens ein Drittel der Inkubationszeit betragen.

Die Ausbreitungsgeschwindigkeit in der Fixierungsschicht wird im wesentlichen von ihrer Saugkraft und von dem in der Schicht herrschenden Strömungswiderstand beeinflußt. Die Saugkraft ist von den Eigenschaften der Materialoberfläche und von der Kapillargröße abhängig. Die Oberflächenspannung der Materialoberfläche sollte so sein, daß das Material von der Flüssigkeit benetzt wird, d.h. hydrophil ist. Bei gegebener Oberflächenspannung ist die Saugkraft umso grösser, je kleiner die Kapillarspalten sind. Andererseits nimmt jedoch der Strömungswiderstand zu, wenn die Kapillarspalten in der Fixierungsschicht enger werden. Der Fachmann kann aufgrund dieser allgemeinen Hinweise aus der Vielzahl der bekannten porösen Schichtmaterialien ein geeignetes Material ausreichend hoher Ausbreitungsgeschwindigkeit auswählen.

Wie dargelegt, hängt die Ausbreitungsgeschwindigkeit in der Fixierungsschicht nicht alleine von deren Saugkraft ab. Vorzugsweise sollte jedoch auch die Saugkraft für sich genommen verhältnismäßig hoch sein, insbesondere höher als die Saugkraft der Konjugatschicht. Dadurch wird Flüssigkeit, die der Konjugatschicht zugeführt wird, nahezu vollständig und verhältnismäßig schnell in die Fixierungsschicht weitergesaugt, mit der die Konjugatschicht vorzugsweise ständig in Flüssigkeitsaustausch ermöglichendem Kontakt steht.

Die optische Nachweisschicht ist manuell oder mit Hilfe eines Auswertegerätes zwischen der ersten und der zweiten Position bewegbar. Vorzugsweise befindet sie sich in der zweiten Position derartig über oder unter der Fixierungsschicht, daß der Flüssigkeitsaustausch durch die benachbarten Flächen der Schicht senkrecht zu diesen erfolgt. Die Fixierungsschicht kann dabei unmittelbar in Flächenkontakt zur optischen Nachweisschicht stehen oder es können eine oder mehrere poröse Schichten dazwischen vorhanden sein, durch die hindurch der Flüssigkeitsaustausch stattfindet.

Die genannten Anforderungen an die Fixierungsschicht lassen sich mit Hilfe einer Vielzahl verschiedener Schichtstrukturen erfüllen. Geeignet sind beispielsweise poröse Kunststoffmaterialien mit ausreichend großem mittleren Porendurchmesser oder Papiere als Basismaterial für die Fixierungsschicht. Bevorzugst basiert die Fixierungsschicht auf einer Faserverbundstruktur, bei der Fasern oder Fäden so verbunden sind, daß sie als Ausgangsmaterial für eine Testträgerschicht geeignet sind. Wichtige Beispiele sind die bekannten textilen Verbundstrukturen wie Gewebe oder Gewirke.

Besonders bevorzugt sind Schichtstrukturen auf Basis eines Faservlieses. Derartige Vliese haben meist eine Vorzugsrichtung der Faserlage. In dieser Richtung findet eine verhältnismäßig schnelle Ausbreitung statt, obwohl die Materialdichte (und damit die Kapillarkraft) verhältnismäßig hoch ist. Vorzugsweise werden derartige Vliese im Naßverfahren auf hydrodynamischem Weg hergestellt, d.h. das Vlies wird aus einer wässrigen Suspension der entsprechenden Faser verdichtet.

Der in der Fixierungsschicht fixierte Bindungspartner kann unmittelbar an die strukturbildende Komponente der Schicht gebunden sein. Beispielsweise lassen sich Kunststoffmaterialien, aus denen eine poröse Kunststoffschicht, ein Kunststoffgewebe oder ein Kunststoff-Faservlies hergestellt sind, durch Anäzen mit reaktiven organischen Gruppen versehen, an die beispielsweise Antigene oder Antikörper kovalent gebunden werden. Derartige Bindungsverfahren sind dem Fachmann bekannt; es sei beispielsweise auf die DE-A-33 29 728 und die DE-B-34 45 816 verwiesen.

Eine besonders hohe Beladungsdichte des trägerfixierten immunologischen Bindungspartners läßt sich gemäß einer Weiterbildung der Erfindung erreichen, bei der der Bindungspartner an Partikeln fixiert ist, die in die Schichtstruktur der Fixierungsschicht eingebettet sind. Geeignet sind sowohl Kunststoffteilchen (Latexpartikel) als auch Teilchen aus anorganischem Material. Im letztgenannten Fall wird der immunologische Bindungspartner über reaktive organische Gruppen, die zuvor an die anorganische Partikelstruktur angekoppelt wurden, gebunden. Die reaktiven organischen Gruppen können insbesondere Amingruppen oder Carboxylgruppen sein, an die der immunologische Bindungspartner mit Hilfe einer Amidbindung angekoppelt wird. Eine derartige Koppelung für Antikörper wird im Zusammenhang mit der Herstellung von Chromatographiesäulen in mehreren Varianten in der US-A-45 60 504 beschrieben. Nähere Einzelheiten zur Bindung von Immunreagenzien an Trägerpartikel sind auch der deutschen Patentanmeldung 37 40 471, angemeldet am 28.11. 1987, zu entnehmen.

Die Partikel können Teil einer dreidimensionalen Partikelverbundstruktur sein, wie sie beispielsweise in der EP-A 13 156 beschrieben ist. Weitere Beispiele sind in der DE-A-33 29 728 erwähnt.

Besonders bevorzugt sind die Partikel aber in eine Faserverbundstruktur, insbesondere in ein Faservlies eingebettet. Eine derartige Fixierungsschicht wird vorzugsweise in der Weise hergestellt, daß man in einem ersten Verfahrensschritt den immunologischen Bindungspartner an Trägerpartikeln fixiert, in einem zweiten Verfahrensschritt die Partikel von dem zur Fixierung des Bindungspartners nötigen Substanzen (durch Filtrieren, Sedimentieren oder Zentrifugieren) trennt, in einem dritten Verfahrensschritt eine aus einer Faserverbundstruktur bestehende Materialbahn mit einer Suspension der Trägerpartikel tränkt und in einem vierten Verfahrensschritt die Materialbahn trocknet.

Im Rahmen der vorliegenden Erfindung wurde festgestellt, daß die Partikel in einer nach diesem Verfahren hergestellten Fixierungsschicht ausreichend haften, so daß sie und mit ihnen der an ihre Oberfläche gebundene Bindungspartner in der Schichtstruktur fixiert sind. Außerdem zeichnen sich solche Schichten durch hohe Beladungsdichte des immunologischen Bindungspartners , schnelle Saugfähigkeit und geringen Flüssigkeitsbedarf aus.

Die Partikel sollen kleiner sein als die Poren der Struktur, in der sie eingebettet sind. Bevorzugte durchschnittliche Durchmesser sind 0,01 (bevorzugt 0,05) bis 5»m, besonders bevorzugt 0,3 bis 1»m.

Bei einer besonders vorteilhaften Ausführungsform der Erfindung wird ein Glasfasern enthaltendes Vlies als Ausgangsmaterial für die Fixierungsschicht verwendet. Der Glasfasergehalt liegt vorzugsweise bei mindestens 60 %. Auch in diesem Fall ist der immunologische Bindungspartner vorzugsweise an Partikel fixiert, die - vorzugsweise nach dem beschriebenen Tränkungsverfahren - in die Glasfaserschicht eingebettet sind. Ein solches Glasfaservlies zeichnet sich durch gute Transporteigenschaften aus und läßt sich in sehr geringer Schichtdicke herstellen, wobei es zweckmäßigerweise durch Zugabe von Polyvinylalkohol verstärkt ist, wie dies in der EP-A-2 39 002 beschrieben wird. Ein weiterer Vorteil der Glasfasern ist eine verhältnismäßig geringe unspezifische Bindung von Immunreagenzien an der Glasfaseroberfläche. Außerdem sind Glasfasern, wie in der US-A- 4 477 575 beschrieben wird, geeignet, den roten Blutfarbstoff (Erythrozyten) aus Vollblut abzutrennen. Damit lassen sich Testträger herstellen, die Blut ohne vorhergehende Plasma- bzw. Serumgewinnung verarbeiten können.

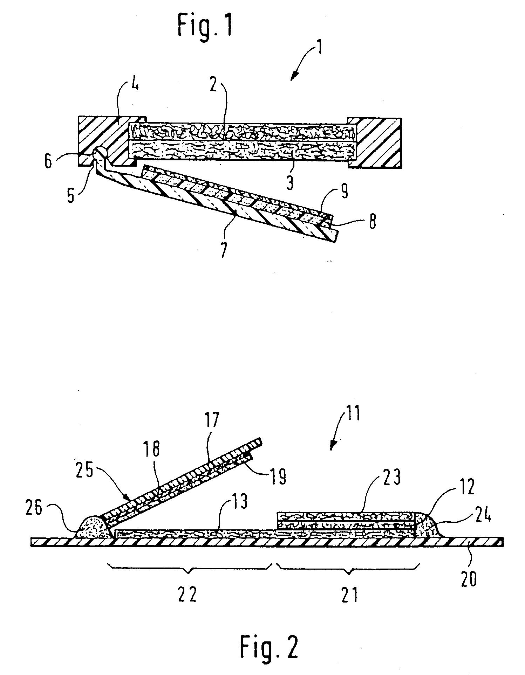

Die Erfindung wird im folgenden anhand von in den Figuren schematisch dargestellen Ausführungsbeispielen näher erläutert; es zeigen:
- Fig. 1: eine erste Ausführungsform eines erfindungsgemäßen Testträgers im Schnitt
- Fig. 2: eine zweite Ausführungsform eines erfindungsgemäßen Testträgers im Schnitt
Bei dem in Figur 1 dargestellten Testträger 1 wird eine Konjugatschicht 2 und eine Fixierungsschicht 3 von einem Rahmen 4 gehalten. Der Rahmen 4 ist vorzugsweise ein Kunststoffspritzteil mit einer Ausnehmung 5, in der eine Achsleiste 6 eines Kunststoffgelenks verläuft. Die Achsleiste 6 ist Bestandteil eines durchsichtigen Kunststoffträgers 7, an dem eine optische Nachweisschicht 8 befestigt ist. Die optische Nachweisschicht ist mit einer optischen Sperrschicht 9 bedeckt.

In der Figur ist eine erste Position der optischen Nachweisschicht 8 dargestellt, in der kein Kontakt zur Fixierungsschicht 3 besteht, so daß kein Flüssigkeitsübertritt möglich ist. Der Kunststoffträger ist jedoch um die Achsleiste 6 klappbar, so daß die optische Sperrschicht 9 in Kontakt mit der Fixierungsschicht 3 kommt. In dieser (nicht dargestellten) zweiten Position ist ein Flüssigkeitsaustausch senkrecht zur Kontaktfläche zwischen der optischen Nachweisschicht 8 und der Fixierungsschicht 3 durch die flüssigkeitsdurchlässige optische Sperrschicht 9 hindurch möglich.

Bei dem in Figur 2 dargestellten Testträger 11 ist die Fixierungsschicht 13 auf einer Basisschicht 20 befestigt, die beispielsweise aus einer steifen Kunststoffolie, wie bei Teststreifen üblich, bestehen kann. Der Testträger hat insgesamt eine langgestreckte Form, ähnlich einem Teststreifen.

In Längsrichtung des Testträgers läßt sich ein Probenaufgabebereich 21 und ein Nachweisbereich 22 unterscheiden, wobei sich die Fixierungsschicht 13 von dem Probenaufgabebereich 21 in den Nachweisbereich 22 erstreckt.

In dem Probenaufgabebereich 21 ist über der Fixierungsschicht 13 eine poröse Konjugatschicht 12 und eine Erythrozytenabtrennschicht 23 vorgesehen. Beide sind mit einem Schmelzkleberstreifen 24 so befestigt, daß sie vollflächig aufeinanderaufliegen, so daß ein Flüssigkeitsaustausch zwischen den Schichten 23,12,13 möglich ist.

Im Nachweisbereich 22 wird die Fixierungsschicht 13 von einer Klappe 25 überspannt, die aus einer durchsichtigen, flexiblen Kunststofftragfolie 17, einer optischen Nachweisschicht 18 und einer optischen Sperrschicht 19 besteht.

Die Klappe 25 wird durch einen Schmelzkleberstreifen 26 derartig festgehalten, daß in der dargestellten ersten Position die optische Nachweisschicht 18 keinen Flüssigkeitsaustausch-Kontakt mit der Fixierungsschicht 13 hat. Die Klappe 25 läßt sich jedoch durch externe Manipulation, beispielsweise manuell oder mit Hilfe eines Geräts nach unten drücken. Ein entsprechendes Gerät ist in der EP-A-129 220 beschrieben. Wenn die Klappe 25 gegen die Fixierungsschicht 13 gedrückt wird, ist ein Flüssigkeitsaustausch zwischen der Fixierungsschicht 13 und der optischen Nachweisschicht 18 möglich (zweite Position).

Die Funktion ist bei beiden Testträgern ähnlich.

Eine Probe, die einen zu bestimmenden Bestandteil (Analyt) enthält, wird mit der Konjugatschicht 2,12 in Kontakt gebracht. Bei Figur 2 erfolgt dies indirekt. Die Probe wird auf die Erythrozytenabtrennschicht 23 aufgegeben. Während sie durch die Schicht 23 hindurch sickert, werden Erythrozyten abgetrennt, so daß im wesentlichen nur Plasma (mit dem Analyten) in die Konjugatschicht 12 gelangt (US-A 4477 575).

Um beispielsweise ein in der Probe enthaltenes freies Antigen oder Hapten Agf (f für "free") zu bestimmen, enthält die Konjugatschicht 12 einen Antikörper Ak für dieses Antigen bzw. Hapten, der mit einem entsprechenden Markierungsenzym konjugiert ist. Das Konjugat AkE wird von der eindringenden Probe gelöst. Dabei beginnt die spezifische Bindungsreaktion, d.h. es bilden sich Komplexe Agf-AkE.

Für die vorliegende Erfindung ist es nicht notwendig, daß die Bindungsreaktion zwischen Agf und AkE weit fortschreitet, bevor beide mit der Probenflüssigkeit in die Fixierungsschicht 3,13 eindringen. Die Fixierungsschicht 3,13 ist vielmehr so ausgelegt, daß sich die Flüssigkeit darin schnell ausbreitet.

Die Fixierungsschicht 13 enthält im dargestellten Beispielsfall ein trägerfixiertes Antigen Agb (b für "bound") das mit dem Antigen in der Probe übereinstimmt bzw. mit diesem analog ist, d.h. mit dem gleichen Antikörper bindet, so daß sich trägerfixierte Komplexe Agb-AkE bilden.

Durch die erfindungsgemäße schnelle Ausbreitung der Flüssigkeit in der Fixierungsschicht 3,13 wird diese überall gleichmäßig benetzt und enthält zu Beginn der Reaktion etwa die gleiche Konzentration der Reaktionskomponenten. Die Schicht 3,13 bildet also praktisch eine Art Küvette für das Reaktionsgemisch, in der die Bindungsreaktion zwischen den beteiligten immunologischen Bindungspartnern räumlich homogen abläuft.

Erst nach Ablauf einer gewünschten, auf die Erfordernisse des jeweiligen Tests abgestimmten Inkubationszeit wird die optische Nachweisschicht 8,18 in die zweite Position und damit in Flüssigkeitskontakt mit der Fixierungsschicht 3,13 gebracht.

Dadurch gelangt die Probenflüssigkeit mit den darin enthaltenen Agf-AkE-Komplexen in die optische Nachweisschicht. Dort erzeugt das Enzym in bekannter Weise einen Farbumschlag des Substrats. Die Geschwindigkeit des Farbumschlags ist charakteristisch für die Konzentration des Enzyms und damit für die Konzentration des Analyten. Dadurch, daß die optische Nachweisschicht 8,18 mit ihrer gesamten Fläche in Flüssigkeitskontakt mit der Fixierungsschicht 3,13 gebracht wird, ergibt sich ein gleichmäßiger Übertritt in die optische Nachweisschicht und damit eine homogene Farbbildung, die durch die durchsichtigen Träger 7,17 hindurch, also auf der von der Fixierungsschicht 3,13 abgewandten Seite der optischen Nachweisschicht 8,18 photometrisch ausgemessen wird.

Da die üblichen farbbildenden Substrate löslich sind, führt das Eindringen der Probenflüssigkeit in die optische Nachweisschicht dazu, daß Substrat auch rückwärts in die Fixierungsschicht gelangen kann. Dadurch ergibt sich eine störende Farbbildung in der Fixierungsschicht aufgrund des dort vorhandenen trägerfixierten Enzyms. Um störende Einflüsse auf die optische Messung zu vermeiden, ist die optische Sperrschicht zwischen der optischen Nachweisschicht und der Fixierungsschicht vorgesehen. Sie besteht beispielsweise aus einer porösen Struktur, die ein Pigment, vorzugsweise Titandioxid, enthält. Ein derartiger zweischichtiger Aufbau aus optischer Sperrschicht 9,19 und optischer Nachweisschicht 8,18 ist in der Testträgertechnik für andere Anwendungsteile bekannt und muß deshalb nicht näher erläutert werden.

Statt des beschriebenen zweischichtigen Aufbaus besteht auch die Möglichkeit, eine optische Nachweisschicht zu verwenden, die selbst eine ausreichend hohe Pigmentkonzentration enthält, um eine Störung der Messung durch die Farbbildung in der Fixierungsschicht zu vermeiden. Auch dies ist eine in der Testträgertechnik an sich bekannte Maßnahme, die im vorliegenden Fall gegenüber dem zweischichtigen Aufbau weniger bevorzugt ist. Der zweischichtige Aufbau unter Verwendung einer dünnen optischen Nachweisschicht führt zu einer kräftigen Farbbildung bei geringem Probenvolumen.

Wie erwähnt, sollte die Saugkraft der Fixierungsschicht 3,13 größer sein als die der Konjugatschicht 2,12. Soweit der Testträger weitere vorgelagerte Schichten aufweist, wie bei Figur 2 die Erythrozytenabtrennschicht 23, sollte auch deren Saugkraft geringer sein als die der Fixierungsschicht. Dadurch werden die vorgelagerten Schichten von der Fixierungsschicht weitgehend leergesaugt, und es ist möglich, mit einem besonders geringen Probenvolumen zu arbeiten.

Die Erfindung ist auch für andere als das hier im einzelnen beschriebene Analyseverfahren geeignet. So muß beispielsweise das freie Antigen Agf nicht selbst der Analyt sein. Vielmehr sind Testführungen möglich, bei denen vorgelagerte Reaktionen zur Bildung einer Konzentration an freiem Antigen führen, die ihrerseits für die Konzentration des ursprünglichen Analyten charakteristisch ist. Selbstverständlich ist auch ein Testablauf möglich, bei dem jeweils Antigene und Antikörper vertauscht sind, bei dem also zum Nachweis eines freien Antikörpers ein Antigenkonjugat in der Konjugatschicht und ein trägerfixierter Antikörper in der Fixierungsschicht verwendet werden. Die Menge der Reagenzien in den Schichten ist von den spezifischen Besonderheiten des Tests abhängig, wobei bezüglich der immunologischen Reaktionsbestandteile, insbesondere die Affinität der Bindungspartner zueinander eine Rolle spielt. Sie wird daher -wie üblich- experimentell optimiert.

Die in Figur 2 dargestellte Ausführungsform ist gegenüber der in Figur 1 dargestellten Ausführungsform bevorzugt, weil sie eine besonders einfache Herstellung ermöglicht. In der Fixierungsschicht findet ein Längstransport der Flüssigkeit parallel zu den Schichtoberflächen statt, der zugleich benutzt werden kann, um Erythrozyten abzutrennen. Der Aufbau erlaubt es, mit sehr dünnen Schichten zu arbeiten, so daß insgesamt Analysen mit einem sehr geringen Probenvolumen von beispielsweise 30 »l möglich sind. Die Fixierungsschicht ist vorzugsweise 50 bis 500 »m, besonders bevorzugt weniger als 300 »m dick.

### Beispiele

### 1. Theophyllin-Test

Ein Testträger mit dem prinzipiellen Aufbau gemäß Figur 2 wurde wie folgt hergestellt:

### Herstellung der Konjugatschicht 12:

Anti-Theophyllin-AK, konjugiert mit β-Galaktosidase wird in phosphate buffered saline (PBS) mit 1% Crotein® C (Croda GmbH, Nettetal, Bundesrepublik Deutschland (BRD)) auf Schablonenpapier (Schoeller u. Hoesch, Gernsbach, BRD,) getränkt, so daß 1U Enzymaktivität pro cm² zur Verfügung steht.

### Herstellung der Fixierungsschicht 13:

Zunächst werden Latex-Partikel mit einem Analyt-Analogon wie folgt beladen: Eine 10%ige stabilisierte Suspension von kalibriertem Latex (Durchmesser 0,66 »m, Rhone-Poulenc, Aubervilliers, Frankreich) wird durch eine G5-Fritte unter Vakuum abgesaugt. Die vor der Fritte zurückbleibenden Latexpartikel werden in H₂O dest.resuspendiert und mit 25 mg/g Latex N-Ethyl-Morpholino-Carbodiimid versetzt. Nach 30 min. werden 20mg Polyhapten pro Gramm Latex über Nacht inkubiert. Anfänglich wird der pH überprüft und auf etwa 5 eingestellt. Als Polyhapten wird dabei ein unspezifischer Antikörper, an den kovalent nach bekannten Verfahren Theophyllin gekoppelt ist, verwendet. Dabei ist das Linker-Molekül so ausgewählt, daß entsprechend der DE-A-36 24 464 die Affinität des eingesetzten Antikörpers zum Polyhapten erheblich höher ist als zum freien Theophyllin. Man erreicht eine Beladung von etwa 4 »g Polyhapten/g Latex.

Die so hergestellten Polyhapten-beladenen Latexpartikel werden 10%ig in PBS (+ 0,05% Detergens "Brij® 58", Serva, Heidelberg, BRD) suspendiert. Mit der Suspension wird ein Glasfaservlies getränkt, das wie folgt hergestellt wird:
Als Ausgangsmaterialien dienen:
1000 1 dest. Wasser
1,0 kg Glasfaser Type 108 A (John Mansville, Denver/USA)
0,05 kg Polyvinylalkohol Typ Kuralon® VPB 105-2 (Rohtext Textil GmbH,Mönchengladbach,BRD)
Zur Herstellung des Vlies wird eine Schrägsiebmaschine verwendet. Die Fasermaterialien werden als 0,1% Mischung aufgeschlagen. Der Faserstoff wird auf ein Schrägsieb gepumpt. Während die Flüssigkeit abfließt bzw. durch Vakuum abgesaugt wird, orientieren sich die Fasern auf der Sieboberfläche und werden als Vlies über Trockenzylinder getrocknet. Die Trocknung findet bei 125^{o} statt, bis eine Endfeuchte von 0,5 bis 1,5 Gew.% erreicht ist. Die Absaug- und die Transportgeschwindigkeit werden so gewählt, daß ein Material eines Flächengewichts von 30 g/m² und einer Dicke von 0,25 mm entsteht.

### Herstellung des Erythrozytenabtrennvlieses 23:

Das Erythrozythenabtrennvlies wird ebenso wie das als Basis der Fixierungsschicht dienende Glasfaservlies hergestellt.

### Herstellung der optischen Nachweisschicht 18 und der optischen Sperrschicht 19 im Verbund:

Eine Beschichtungsmasse mit folgender Zusammensetzung wird hergestellt:

| | |
|---|---|
| Propiofan® 70 D (BASF Ludwigshafen/BRD) | 17 g |
| Texamid 578 (Kelco, Hamburg/BRD) 83, 1%ig | 1,44 g |
| Kieselgur | 11 g |
| Chlorphenolrotgalactosid (Boehringer Mannheim/BRD) | 0,88 g |
| Puffer (PBS mit 5 mM MgC1₂) | 68,5 g |
| | 100 g |

Diese Beschichtungsmasse wird auf eine Basisschicht aus Pokalon® (Schichtdicke 200 »m, Lonza) aufgetragen und mit einer Naßfilmdicke von 150 »m ausgestrichen ("gerakelt").

Die Beschichtungsmasse für den optischen Sperrfilm enthält folgende Bestandteile:

| | |
|---|---|
| Propiofan® 70 D (BASF Ludwigshafen,BRD) | 19,0 g |
| Texamid 578 (83,1%ig) | 0,5 g |
| Kieselgur | 36,1 g |
| TiO₂ RN 56 (Kronos AG, Leverkusen/BRD) | 7,5 g |
| PBS (mit 5 mM MgC1₂) | 76,0 g |
| Triton® X 100 (Serva) | 0,3 g |
| | 1̅4̅0̅,̅0̅ g̅ |

Die Beschichtungsmasse wird auf den zuvor bei 50^{o} C für 45 min. getrockneten optischen Nachweisfilm aufgetragen und mit 250 »m Naßfilmdicke ausgestrichen. Die Trocknung erfolgt bei den gleichen Bedingungen wie zuvor.

Die Bestandteile des Testträgers haben etwa folgende Abmessungen:

| | |
|---|---|
| Erythrozytenabtrennschicht: | 6 x 6 mm |
| Konjugatschicht: | 7 x 6 mm |
| Fixierungsschicht: | 17 x 6 mm |
| Klappe 25: | 15 x 6 mm |

### Nachweis von Theophyllin

30 »l Serum mit verschiedenen Theophyllinkonzentrationen werden aufgetragen. Die Streifen werden in dem Gerät "Reflotron®" der Anmelderin eingelegt. Von der Aufgabe der Probe bis zur vollständigen Ausbreitung auf der Fixierungsschicht vergehen maximal etwa 30 sec. Nach etwa 180 sec. ist die Inkubationszeit der immunologischen Bindungspartner abgelaufen,und die Klappe 25 wird gegen die Fixierungsschicht 13 gedrückt. Nach etwa 230 sec. wird die Remission mit einer Wellenlänge (λ = 567 nm) gemessen. Dabei ergeben sich folgende Werte:

| c (Theo) /mg/l | % R |
|---|---|
| 0 | 55,3 |
| 2 | 51,0 |
| 6 | 47,5 |
| 10 | 44,0 |
| 24 | 39,5 |
| 30 | 37 |
| 45 | 35 |

Die erreichte Abstufung im klinisch relevanten Meßbereich von Theophyllin (2 - 30 mg/l) ist völlig ausreichend, um eine genaue Bestimmung des Parameters zu ermöglichen.

### 2. T-Uptake-Test

### Herstellung der Konjugatschicht

Die Konjugatschicht enthält in diesem Fall T4, konjugiert mit β-Galaktosidase. Im übrigen ist die Schichtherstellung analog Beispiel 1, wobei eine Enzymaktivität von 0,1 U/cm² eingestellt wird.

### Herstellung der Fixierungsschicht

Die Fixierungsschicht wird analog Beispiel 1 hergestellt, wobei jedoch anorganische TiO₂-Partikel als Träger für den trägerfixierten zweiten Bindungspartner verwendet wird:
10 g TiO₂ (RN 43,Kronos) werden in 400 ml Wasser, welches mit Essigsäure auf pH 3,5 eingestellt ist und mit 4 ml GFZO-Silan (3-Triethoxysilylpropyl)-bernsteinsäure-anhydrid, Wacker-Chemie, München/BRD) für 2 h gerührt. Danach wird die Flüssigkeit durch eine Fritte unter Vakuum abgesaugt. Es folgt eine mehrfache Waschung mit H₂O und eine Trocknung von einer Stunde bei 140^{o} C.

An das so hergestellte COOH-modifizierte TiO₂ wird anti-TBG-Antikörper analog zu Beispiel 1 über Carbodiimid gekoppelt (5 mg AK/g TiO₂). Das Antikörper-beladene TiO₂ wird 25% (w/v) in das analog zu Beispiel 1 hergestellte Glasfaservlies getränkt.

Die optische Nachweisschicht 18 und die optische Sperrschicht 19 stimmen mit Beispiel 1 überein.

### T4-Uptake Analyse

30 »l Probe (Serum) mit verschiedenen TBG-Konzentrationen werden aufgetragen; der Streifen wird, wie bei Beispiel 1, in ein "Reflotron"-Gerät gelegt. Die Füllung der Fixierungsschicht ist in diesem Fall nach spätestens 30 sec. abgeschlossen. Das Schließen der Klappe erfolgt nach 150 sec. Die Remissionsmessung erfolgt bei λ = 567 nm nach 230 sec. Es werden folgende Meßergebnisse erhalten:

| TBG-Probe | % R |
|---|---|
| Puffer (= 0 TBG) | 29,8 |
| "TBG-freies Serum" ( ≦ 0,05 »mol/l | 33,2 |
| Normalserum (ca. 0,25»mol/l) | 42,2 |
| Pathologisch erhöhtes Serum (ca. 0,42 »mol/l | 50,8 |

Auch in diesem Fall wird eine sehr gute Abstufung von sehr geringen TBG-Werten bis zu erhöhten TBG-Werten erreicht.

## Patentansprüche

1. Testträger für die analytische Bestimmung eines Bestandteils einer Probenflüssigkeit mit Hilfe von immunologischen Reagenzien mit
einer Konjugatschicht (2,12), die ein lösliches Konjugat eines ersten immunologischen Bindungspartners mit einer Markierung enthält,
einer porösen Fixierungsschicht (3,13), die einen mit dem ersten Bindungspartner spezifisch bindungsfähigen zweiten Bindungspartner in trägerfixierter Form enthält und
einer optischen Nachweisschicht (8,18)
**dadurch gekennzeichnet,** daß
die Fixierungsschicht (3,13) derart ausgebildet ist, daß die Sauggeschwindigkeit, mit der die Flüssigkeit in ihr transportiert wird, so hoch ist, daß die zur Ausbreitung der Flüssigkeit in der Fixierungsschicht benötigte Zeit erheblich kürzer ist als die Inkubationszeit, die zum Ablauf der spezifischen Bindungsreaktion zwischen dem ersten Bindungspartner und dem zweiten Bindungspartner nötig ist und
die optische Nachweisschicht (8,18) an dem Testträger (1,11) so befestigt ist, daß sie in einer ersten Position nicht in Flüssigkeitsaustausch ermöglichendem Kontakt zu der Fixierungsschicht (3,13) steht, jedoch in eine zweite Position bringbar ist, in der ein Flüssigkeitsaustausch zwischen der Fixierungsschicht (3,13) und der optischen Nachweisschicht (8,18) möglich ist.

2. Testträger nach Anspruch 1, **dadurch gekennzeichnet,** daß die optische Nachweisschicht (8,18) in der zweiten Position derart über oder unter der Fixierungsschicht angeordnet ist, daß der Flüssigkeitsaustausch durch die benachbarten Oberflächen der Schichten hindurch senkrecht zu diesen erfolgt.

3. Testträger nach Anspruch 1, **dadurch gekennzeichnet,** daß die Saugkraft der Fixierungsschicht (3,13) größer ist als die Saugkraft der Konjugatschicht (2,12).

4. Testträger nach Anspruch 1, **dadurch gekennzeichnet,** daß die optische Nachweisschicht (8,18) auf der der Fixierungsschicht (3,13) zugewandten Seite mit einer optischen Sperrschicht (9,19) verbunden ist, die für die Flüssigkeit durchlässig ist, so daß der Flüssigkeitsaustausch in der zweiten Position durch die optische Sperrschicht (9,19) hindurch erfolgt.

5. Testträger nach Anspruch 1, **dadurch gekennzeichnet,** daß das Ausgangsmaterial der Fixierungsschicht (3,13) eine Faserverbundstruktur hat.

6. Testträger nach Anspruch 5, **dadurch gekennzeichnet,** daß die Faserverbundstruktur (3,13) eine Vliesstruktur ist.

7. Testträger nach Anspruch 5, **dadurch gekennzeichnet,** daß die Faserverbundstruktur Kunststoffasern enthält.

8. Testträger nach Anspruch 5, **dadurch gekennzeichnet,** daß die Faserverbundstruktur Glasfasern enthält.

9. Testträger nach Anspruch 8, **dadurch gekennzeichnet,** daß die Faserverbundstruktur Polyvinylalkohol enthält.

10. Testträger nach Anspruch 1 oder 5, **dadurch gekennzeichnet,** daß der zweite Bindungspartner an Partikeln fixiert ist, die in die Schichtstruktur der Fixierungsschicht (3,13) eingebettet sind.

11. Testträger nach Anspruch 10, **dadurch gekennzeichnet,** daß die Partikel aus einem Kunststoffmaterial bestehen.

12. Testträger nach Anspruch 10, **dadurch gekennzeichnet,** daß die Partikel aus einem anorganischen Material bestehen.

13. Testträger nach Anspruch 10, **dadurch gekennzeichnet,** daß der mittlere Partikeldurchmesser 0,01 bis 5 »m, bevorzugt 0,3 bis 1 »m beträgt.

14. Testträger nach Anspruch 1, **dadurch gekennzeichnet,** daß eine Basisschicht (20) vorgesehen ist, an der die Konjugatschicht, die Fixierungsschicht und die optische Nachweisschicht befestigt sind, wobei
die Fixierungsschicht (13) parallel zu der Basisschicht (20) von einem Probenaufgabebereich (21) in einen Auswertebereich (22) verläuft,
die Konjugatschicht in dem Probenaufgabebereich (21) angeordnet ist und
die optische Nachweisschicht (18) in dem Auswertebereich (22) angeordnet ist und die Fixierungsschicht (13) klappenartig überspannt.

15. Verfahren zur immunologischen analytischen Bestimmung eines Bestandteils einer Probenflüssigkeit mit Hilfe eines Testträgers
bei dem ein Konjugat eines ersten immunologischen Bindungspartners mit einer Markierung, das in einer Konjugatschicht des Testträgers enthalten ist, in der Probenflüssigkeit aufgelöst wird,
die Probenflüssigkeit mit dem Konjugat des ersten immunologischen Bindungspartners in eine poröse Fixierungsschicht übertritt, die einen mit dem ersten Bindungspartner spezifisch bindungsfähigen zweiten Bindungspartner in trägerfixierter Form enthält, wobei eine spezifische Bindungsreaktion zwischen dem ersten Bindungspartner und dem zweiten Bindungspartner stattfindet und ein Teil des Konjugats fixiert wird und
die Probenflüssigkeit mit nichtfixiertem Konjugat in eine optische Nachweisschicht übertritt, in der eine für die Menge des nichtfixierten, in die optische Nachweisschicht übergetretenen Konjugats charakteristische, optisch nachweisbare Veränderung stattfindet,
**dadurch gekennzeichnet,** daß
die Ausbreitung der Probenflüssigkeit in der Fixierungsschicht in erheblich kürzerer Zeit erfolgt als die Inkubationszeit der spezifischen Bindungsreaktion und
die optische Nachweisschicht sich während der Inkubationszeit nicht in Flüssigkeitskontakt zu der Fixierungsschicht befindet, jedoch nach Ablauf der Inkubationszeit in Flüssigkeitskontakt mit der Fixierungsschicht gebracht wird, wonach die Flüssigkeit von der Fixierungsschicht in die optische Nachweisschicht übertritt.

16. Verfahren nach Anspruch 15, **dadurch gekennzeichnet,** daß die Flüssigkeit in der Fixierungsschicht parallel zu deren Oberflächen strömt.

17. Verfahren zur Herstellung einer einen trägerfixierten immunologischen Bindungspartner enthaltenden Fixierungsschicht für Testträger nach Anspruch 1
**dadurch gekennzeichnet,** daß man
in einem ersten Verfahrensschritt den immunologischen Bindungspartner an Trägerpartikel fixiert,
in einem zweiten Verfahrensschritt die Partikel von den zur Fixierung des Bindungspartners nötigen Substanzen trennt,
in einem dritten Verfahrensschritt eine aus einer Faserverbundstruktur bestehende Materialbahn mit einer Suspension der Trägerpartikel tränkt und
in einem vierten Verfahrensschritt die Materialbahn trocknet.

## Claims

1. Test carrier for the analytical determination of a component of a sample liquid with the help of immunological reagents with a conjugate layer (2,12), which contains a soluble conjugate of a first immunological binding partner with a label, a porous fixing layer (3,13), which contains a second binding partner in carrier-fixed form binding specifically with the first binding partner, and an optical detection layer (8,18), characterised in that the fixing layer (3,13) is constructed in such a manner that the suction speed with which the liquid is transported in it is so high that the time needed for the spreading out of the liquid in the filing layer is considerably shorter than the incubation time which is needed for the completion of the specific binding reaction between the first binding partner and the second binding partner and the optical detection layer (8,18) is so fixed on the test carrier (1,11) that, in a first position, it is not in contact with the fixing layer (3,13) making possible a liquid exchange but can be brought into a second position in which a liquid exchange between the fixing layer (3,13) and the optical detection layer (8,18) is possible.

2. Test carrier according to claim 1, characterised in that the optical detection layer (8,18) in the second position is arranged above or below the fixing layer in such a manner that the liquid exchange takes place through the neighbouring surfaces of the layers perpendicular to these.

3. Test carrier according to claim 1, characterised in that the suction force of the fixing layer (3,13) is greater than the suction force of the conjugate layer (2,12).

4. Test carrier according to claim 1, characterised in that the optical detection layer (8,18) is connected on the side facing the fixing layer (3,13) with an optical barrier layer (9,19) which is permeable for the liquid so that the liquid exchange in the second position takes place through the optical barrier layer (9,19).

5. Test carrier according to claim 1, characterised in that the starting material of the fixing layer (3,13) has a fibre composite structure.

6. Test carrier according to claim 5, characterised in that the fibre composite structure (3,13) is a fleece structure.

7. Test carrier according to claim 5, characterised in that the fibre composite structure contains synthetic material fibres.

8. Test carrier according to claim 5, characterised in that the fibre composite structure contains glass fibres.

9. Test carrier according to claim 8, characterised in that the fibre composite structure contains polyvinyl alcohol.

10. Test carrier according to claim 1 or 5, characterised in that the second binding partner is fixed on particles which are embedded in the layer structure of the fixing layer (3,13).

11. Test carrier according to claim 10, characterised in that the particles consist of a synthetic material.

12. Test carrier according to claim 10, characterised in that the particles consist of an inorganic material.

13. Test carrier according to claim 10, characterised in that the average particle diameter amounts to 0.01 to 5 »m, preferably 0.3 to 1 »m.

14. Test carrier according to claim 1, characterised in that a base layer (20) is provided to which are fixed the conjugate layer, the fixing layer and the optical detection layer, whereby the fixing layer (13) runs parallel to the base layer (20) from a sample application region (21) into an evaluation region (22), the conjugate layer is arranged in the sample application region (21) and the optical detection region (18) is arranged in the evaluation region (22) and bridges the fixing layer (13) in the manner of a flap.

15. Process for the immunological analytical determination of a component of a sample liquid with the help of a test carrier in which a conjugate of a first immunological binding partner with a label, which is contained in a conjugate layer of the test carrier, is dissolved in the sample liquid, the sample liquid with the conjugate of the first immunological binding partner passes over into a porous fixing layer, which contains in carrier-fixed form a second binding partner specifically bindable with the first binding partner, whereby a specific binding reaction takes place between the first binding partner and the second binding partner and a part of the conjugate is fixed and the sample liquid with non-fixed conjugate passes over into an optical detection layer in which an optically detectable change takes place characteristic for the amount of non-fixed conjugate passing over into the optical detection layer, characterised in that the spreading out of the sample liquid in the fixing layer takes place in a considerably shorter time than the incubation time of the specific binding reaction and the optical detection layer is not in liquid contact with the filing layer during the incubation time but, after completion of the incubation time, is brought into liquid contact with the fixing layer, whereafter the liquid passes over from the fixing layer into the optical detection layer.

16. Process according to claim 15, characterised in that the liquid runs in the fixing layer parallel to its surface.

17. Process for the production of a fixing layer for test carriers according to claim 1 containing a carrier-fixed immunological binding partner, characterised in that, in a first process step, one fixes the immunological binding partner to carrier particles, in a second process step separates the particles from the substances needed for the fixing of the binding partner, in a third process step, impregnates a material strip consisting of a fibre composite structure with a suspension of the carrier particles and, in a fourth process step, dries the material strip.

## Revendications

1. Support de test pour la détermination analytique d'un constituant d'un échantillon liquide à l'aide de réactifs immunologiques, comprenant une couche de conjugué (2,12), contenant un conjugué soluble d'un premier partenaire de liaison immunologique avec un marqueur,
une couche de fixation (3,13) poreuse, contenant un second partenaire de liaison capable de former une liaison spécifique avec le premier partenaire de liaison, sous forme fixée au support, et
une couche de détection optique (8,18), caractérisé en ce que
la couche de fixation (3,13) est conçue de telle façon que la vitesse d'aspiration permettant de transporter le liquide à l'intérieur de ladite couche soit suffisamment élevée pour que le temps nécessaire à la propagation du liquide dans la couche de fixation soit nettement plus court que le temps d'incubation nécessaire au déroulement de la réaction de liaison spécifique entre le premier partenaire de liaison et le second partenaire de liaison, et
la couche de détection optique (8,18) est fixée sur le support de test (1,11) de façon que dans une première position, elle soit en contact avec la couche de fixation (3,13) sans qu'un échange de liquide soit possible, mais elle peut être amenée dans une deuxième position, dans laquelle un échange de liquide entre la couche de fixation (3,13) et la couche de détection optique (8,18) est possible.

2. Support de test selon la revendication 1, caractérisé en ce que dans la deuxième position, la couche de détection optique (8,18) est disposée au-dessus ou en dessous de la couche de fixation de façon que l'échange de liquide s'effectue à travers les surfaces adjacentes des couches, en direction perpendiculaire à celles-ci.

3. Support de test selon la revendication 1, caractérisé en ce que la force d'aspiration dans la couche de fixation (3,13) est plus grande que la force d'aspiration dans la couche de conjugué (2,12).

4. Support de [est selon la revendication 1, caractérisé en ce que la couche de détection optique (8,18) est reliée, sur sa face tournée vers la couche de fixation (3,13), à une couche de barrière optique (9,19) qui est perméable aux liquides, de sorte que l'échange de liquide s'effectue dans la deuxième position à travers la couche de barrière optique (9,19).

5. Support de test selon la revendication 1, caractérisé en ce que le matériau constituant la couche de fixation (3,13) présente une structure composite à base de fibres.

6. Support de test selon la revendication 5, caractérisé en ce que la structure composite à base de fibres (3,13) est une structure non tissée.

7. Support de test selon la revendication 5, caractérisé en ce que la structure composite à base de fibres contient des fibres synthétiques.

8. Support de test selon la revendication 5, caractérisé en ce que la structure composite à base de fibres contient des fibres de verre.

9. Support de test selon la revendication 8, caractérisé en ce que la structure composite à base de fibres contient du poly(alcool vinylique).

10. Support de test selon la revendication 1 ou 5, caractérisé en ce que le second partenaire de liaison est fixé sur des particules qui sont noyées dans la structure de la couche de fixation (3,13).

11. Support de test selon la revendication 10, caractérisé en ce que les particules sont en matière synthétique.

12. Support de test selon la revendication 10, caractérisé en ce que les particules sont constituées d'un matériau inorganique.

13. Support de test selon la revendication 10, caractérisé en ce que le diamètre moyen des particules est de 0,01 à 5 »m, de préférence 0,3 à 1 »m.

14. Support de test selon la revendication 1, caractérisé en ce qu'il est prévu une couche de base (20) sur laquelle sont fixées la couche de conjugué, la couche de fixation et la couche de détection optique, la couche de fixation (13) s'étendant parallèlement à la couche de base (20) depuis une zone d'application de l'échantillon (21) jusqu'à une zone d'évaluation (22), la couche de conjugué étant disposée dans la zone d'application de l'échantillon (21) et la couche de détection optique (18) étant disposée dans la zone d'évaluation (22) et s'étendant au-dessus de la couche de fixation (13) à la manière d'un rabattant.

15. Procédé de détermination analytique immunologique d'un constituant d'un échantillon liquide, au moyen d'un support de test, dans lequel un conjugué d'un premier partenaire de liaison immunologique avec un marqueur, qui est contenu dans une couche de conjugué du support de test, est dissous dans l'échantillon liquide, l'échantillon liquide passe, avec le conjugué du premier partenaire de liaison immunologique, dans une couche de fixation poreuse, qui contient, sous forme fixée au support, un second partenaire de liaison capable de former une liaison spécifique avec le premier partenaire de liaison, une réaction de liaison spécifique ayant lieu entre le premier partenaire de liaison et le second partenaire de liaison, et une partie du conjugué est fixée, et l'échantillon liquide passe, avec le conjugué non fixé, dans une couche de détection optique, dans laquelle se produit une variation optiquement détectable caractéristique de la quantité de conjugué non fixé qui est passée dans la couche de détection optique, caractérisé en ce que la propagation de l'échantillon liquide dans la couche de fixation s'effectue en un laps de temps nettement plus court que le temps d'incubation de la réaction de liaison spécifique, et la couche de détection optique ne se trouve pas en contact d'échange de liquide avec la couche de fixation pendant la période d'incubation, mais après l'écoulement de la période d'incubation, elle est amenée en contact d'échange de liquide avec la couche de fixation, après quoi le liquide passe de la couche de fixation dans la couche de détection optique.

16. Procédé selon la revendication 15, caractérisé en ce que le liquide s'écoule, dans la couche de fixation, parallèlement à la surface de celle-ci.

17. Procédé de préparation d'une couche de fixation pour support de test, contenant un partenaire de liaison immunologique fixé à un support, selon la revendication 1, caractérisé en ce que
dans une première étape du procédé, on fixe le partenaire de liaison immunologique sur des particules de support,
dans une deuxième étape du procédé, on sépare les particules de la substance nécessaire pour la fixation du partenaire de liaison,
dans une troisième étape du procédé, on imprègne une bande de matériau constitué d'une structure composite à base de fibres avec une suspension des particules de support, et
dans une quatrième étape du procédé, on sèche la bande de matériau.
